# EUROPÄISCHE PATENTSCHRIFT

(11) **EP 2 335 747 B1**
(45) Veröffentlichungstag und Bekanntmachung des Hinweises auf die Patenterteilung: **14.12.2016**
(21) Anmeldenummer: 09015561.5
(22) Anmeldetag: 16.12.2009
(51) Int. Cl.: A61M 1/00, C08J 9/02, C08L 83/04

(54) **Vorrichtung zur Unterdrucktherapie von Wunden**
Device for negative pressure wound therapy
Dispositif de traitement de plaies par pression négative

(43) Veröffentlichungstag der Anmeldung: 22.06.2011
(73) Patentinhaber: Paul Hartmann AG, 89522 Heidenheim (DE)
(72) Erfinder: Croizat, Pierre, 89542 Herbrechtingen (DE); Eckstein, Axel, 89522 Heidenheim (DE); Fink, Ulrich, 89522 Heidenheim (DE)
(74) Vertreter: Ter Meer Steinmeister & Partner

(56) Entgegenhaltungen:
- WO-A1-2007/141250
- US-A1- 2007 185 426
- US-A1- 2008 114 276
- US-A1- 2009 227 969

## Beschreibung

Die Erfindung betrifft eine Vorrichtung zur Unterdrucktherapie von Wunden, umfassend ein Abdeckmaterial zum luftdichten Verschließen der Wunde und der Wundumgebung; ein Mittel, geeignet zur Herstellung von Unterdruck im Wundraum, insbesondere zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind; und einen offenzelligen Schaumstoff auf Basis eines quervernetzten Polyorganosiloxans als Wundauflage.

Unter einer Wunde wird die Trennung des Zusammenhangs von Geweben der Körperhülle bei Menschen oder Tieren verstanden. Sie kann mit einem Verlust an Substanz verbunden sein.

Vorrichtungen zur Unterdrucktherapie von Wunden sind im Stand der Technik bekannt.

So beschreibt beispielsweise die WO 1993/009727 A1 eine Vorrichtung zur Förderung der Wundheilung durch die Applikation von Unterdruck auf dem die Wunde aufweisenden und die Wunde umgebenden Hautbereich. Die Vorrichtung gemäß WO 1993/009727 A1 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung. Bei der Wundauflage handelt es sich vorzugsweise um einen offenzelligen Polymer-Schaumstoff, beispielsweise um Polyester-Schaum.

Neben der Verwendung von offenzelligem Polymer-Schaumstoff wurden andere Materialien zur Herstellung von Wundauflagen für die Unterdrucktherapie von Wunden beschrieben.

WO 2001/89431 A1 beschreibt eine Kollagen-Matrix als Wundauflage für die Unterdrucktherapie von Wunden.

GB 2 415 908 beschreibt die Verwendung faserförmigen Materials, welches auch bioresorbierbar sein kann, in Wundauflagen für die Unterdrucktherapie von Wunden.

WO 2006/52839 A1 beschreibt die Verwendung trockenen, faserförmigen Materials oder einer faserförmigen Mischung als Wundkontaktschicht für die Unterdrucktherapie von Wunden. Während der Unterdrucktherapie bildet das faserförmige Material durch Aufnahme von Wundexsudat ein Gel.

WO 2005/123170 A1 offenbart Wundauflagen für die Unterdrucktherapie, mittels derer unerwünschte Substanzen inaktiviert oder aus dem Wundraum entfernt werden sollen und/oder erwünschte Substanzen, welche im Wundraum vorhanden sind, konzentriert werden sollen. Als für den erwünschten Zweck geeignet Bestandteile von Wundauflagen schlägt die WO 2005/123170 eine Vielzahl von zunächst trockenen Polymeren vor, die durch Wasseraufnahme aus dem Wundexsudat ein Gel bilden können.

WO 2006/048246 A1 beschreibt einen Mehrkomponentenverband zur Unterdruckbehandlung von Wunden, welcher ein superabsorbierendes Polymer aufweist. Das superabsorbierende Polymer kann von einer flüssigkeitsdurchlässigen Hülle eingefasst sein und einen trockenen Absorptionskörper bilden, der in den Wundraum eingelegt wird.

US 2007/185426 A1 offenbart eine Vorrichtung zum Anlegen von verringertem Druck für die Unterdruckbehandlung eines Gewebes, wobei die Gewebeauflage mehrere Schichten umfasst, wie die Gewebekontaktschicht, die Trennschicht und die Verteilerschicht Die Trennschicht ist zwischen der Gewebekontaktschicht und der Verteilerschicht angeordnet, um nach Anlegen von vermindertem Druck ein leichtes Lösen der Verteilerschicht von der Gewebekontaktschicht zu ermöglichen.

WO 2007/141250 A1 betrifft schwer entflammbare Organopolysiloxanverbindungen, welche in der Transportindustrie für die Herstellung eines Elastomerschaums (oder Polysilikonschaums) mit geringer Dichte und guten mechanischen Eigenschaften geeignet sind. Die Materialien sind schwer entflammbar und werden als Kabelummantelungen verwendet. Medizinprodukte werden in der Anmeldung nicht erwähnt.

US 2009/227969 A1 offenbart ein System zur Behandlung mit vermindertem Druck, welches eine Unterdruckquelle, eine Zwischenschicht und eine Schicht zum Absorbieren von Flüssigkeiten umfasst.

US 2008/114276 A1 offenbart eine absorbierende Struktur, welche einen Silikonschaumstoff mit Porenwänden und einer Vielzahl an hydrophilen Teilchen umfasst, wobei die hydrophilen Teilchen an den Porenwänden des Silikonschaumstoffes verankert sind. Einige der hydrophilen Teilchen haben einen verankerten Bereich, welcher fest mit den Poren verankert ist, und einen freiliegenden Bereich, der sich nach außen von den Porenwänden, an welchen der verankerte Bereich befestigt ist, erstreckt.

Geräte zur Unterdrucktherapie von Wunden sind kommerziell erhältlich, beispielsweise das V.A.C.^{®}-Gerät der Firma KCl. Bei kommerziell erhältlichen Geräten wird oftmals eine Wundauflage eingesetzt, welche einen offenzelligen Polymer-Schaumstoff, wie beispielsweise Polyvinylalkohol (PVA) oder Polyurethan (PU), enthält.

Die kommerziell üblichen Schaumauflagen werden, abhängig vom angelegten Unterdruck, unterschiedlich stark komprimiert. Dadurch kann eine Verengung der für den Abtransport des Wundexsudats nötigen Durchgänge entstehen. Weiterhin kann bei längerer Anwendung von Polyvinylalkohol oder Polyurethanschaum in der Unterdrucktherapie eine Verklebung des Schaums mit dem Wundgrund auftreten. Verklebter Schaum muss beim Wechseln des Verbandes aufwendig entfernt werden, beispielsweise durch Spülen mit Ringerlösung. In den Schaum eingewachsenes Gewebe kann beim Verbandwechsel zu einer Gewebetraumatisierung und damit zur Störung der Wundheilung führen.

Es hat sich ferner gezeigt, dass die üblichen Polymer-Schaumstoffe aus Polyvinylalkohol (PVA) oder Polyurethan (PU) nur bedingt für längere Wechselintervalle der Wundauflage geeignet sind. Zunächst ist festzustellen, dass die Durchflussrate innerhalb von drei Tagen nicht selten unerwünscht absinkt. Patienten empfinden zudem die üblichen Wundauflagen nicht selten als unangenehm, wenn sie über einen Zeitraum von 3 Tagen ohne Wechsel auf der Wunde verbleiben. Beim Wechsel der Wundauflage nach einem Zeitraum von 3 Tagen oder mehr treten nicht selten unangenehme Gerüche auf. Die Keimdichte ist unerwünscht hoch.

Auch hat sich gezeigt, dass die üblichen Polymer-Schaumstoffe aus Polyvinylalkohol (PVA) oder Polyurethan (PU) teilweise mit der Abdeckfolie verkleben.

Weiterhin hat sich insbesondere bei der Verwendung von PVA-Schaumstoffen gezeigt, dass die Saugkraft in Abhängigkeit vom Port unerwünscht abnimmt. Zudem ist PVA nur in feuchtem Zustand elastisch, was eine Präkonditionierung zwingend erforderlich macht.

Der vorliegenden Erfindung liegt die Aufgabe zugrunde, die Unterdruckbehandlung von Wunden weiter zu verbessern und die Nachteile des Standes der Technik zu überwinden. Es sollen mit der vorliegenden Erfindung Vorrichtungen und Verfahren zur Unterdruckbehandlung von Wunden zur Verfügung gestellt werden, mit denen eine Behandlung möglichst wirksam und möglichst schonend durchgeführt werden kann.

Insbesondere soll die vorliegende Erfindung eine Unterdruckbehandlung von Wunden ermöglichen, die beim Patienten einen Wechsel der Wundauflagen in Intervallen von drei Tagen oder länger ermöglicht. Während eines Intervalls soll die Wundauflage vom Patienten subjektiv als angenehm empfunden werden. Juckreiz und Hautrötungen sollen vermieden werden. Beim Wechsel der Wundauflage nach einem Zeitraum von 3 Tagen oder mehr sollen möglichst wenig unangenehme Gerüche auftreten. Die Keimdichte in der ausgewechselten Wundauflage soll niedrig sein. Ferner soll auch bei längeren Wechselintervallen die Bildung von physiologisch bedenklichen Stoffen, vermieden werden.

Es soll eine Wundauflage bereit gestellt werden, die ein Verkleben mit der Abdeckfolie weitgehend vermeidet. Die Saugkraft soll nicht in Abhängigkeit vom Port abnehmen.

Die Aufgaben konnten unerwartet durch die Verwendung einer Wundauflage, die einen offenzelligen Schaumstoff auf Basis eines quervernetzten Polyorganosiloxans enthält, gelöst werden. Überraschend wurde gefunden, dass eine Vorrichtung zur Unterdrucktherapie umfassend mindestens eine Wundauflage, wobei die Wundauflage einen offenzelligen Schaumstoff auf Basis eines quervernetzten Polyorganosiloxans enthält, zu einer vorteilhaften, d.h. sehr wirksamen und sehr schonenden Behandlung von Wunden, besonders geeignet ist.

Die Erfindung löst somit die Aufgabe mit einer Vorrichtung zur Unterdrucktherapie von Wunden mit den Merkmalen von Anspruch 1.

Gegenstand der Erfindung ist daher eine Vorrichtung zur Unterdrucktherapie von Wunden umfassend,
(a) ein Abdeckmaterial zum luftdichten Verschließen der Wunde und der Wundumgebung,
(b) ein Mittel zur Herstellung von Unterdruck im Wundraum, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind,
(c) einen offenzelligen Schaumstoff auf Basis eines quervernetzten Polyorganosiloxans als Wundauflage.

Schließlich ist Gegenstand der Erfindung ein gebrauchsfertiges Set zur Unterdruck-Wundbehandlung umfassend,
(a) Abdeckmaterial zum luftdichten Verschließen der Wunde und der Wundumgebung,
(b) ein Mittel zur Herstellung von Unterdruck im Wundraum, bevorzugt ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind,
c) einen offenzelligen Schaumstoff auf Basis eines quervernetzten Polyorganosiloxans, wobei der Schaumstoff als Wundauflage geeignet ist und gebrauchsfertig abgepackt vorliegt.

Die neue erfindungsgemäße Vorrichtung zeichnet sich insbesondere dadurch aus, dass sie mindestens eine Wundauflage, enthaltend einen offenzelligen Schaumstoff auf Basis eines quervernetzten Polyorganosiloxans, umfasst. Ein Vorteil des offenzelligen Schaumstoffs auf Basis eines quervernetzten Polyorganosiloxans als Bestandteil der erfindungsgemäßen Vorrichtung besteht darin, dass er eine weiche Wundauflage für die Unterdrucktherapie von Wunden bereitstellt.

Ein weiterer Vorteil des offenzelligen Schaumstoffs auf Basis eines quervernetzten Polyorganosiloxans als Bestandteil der erfindungsgemäßen Vorrichtung besteht darin, dass eine gleichmäßige Druckverteilung auf den Wundgrund gewährleistet werden kann. Durch die Bereitstellung einer weichen Wundauflage und durch die gleichmäßige Druckverteilung kann die Unterdruckbehandlung schonend und besonders wirksam durchgeführt werden.

Ein weiterer Vorteil des offenzelligen Schaumstoffs auf Basis eines quervernetzten Polyorganosiloxans als Bestandteil der erfindungsgemäßen Vorrichtung besteht darin, dass ein Verkleben und/oder Verwachsen des Wundgrundes mit der Wundauflage selbst über einen Zeitraum von 3 Tagen oder mehr weitgehend vermieden werden kann. Eine Traumatisierung der Wunde beim Verbandswechsel kann vermieden werden. Dies erhöht die Wirksamkeit der Wundbehandlung.

Ein weiterer Vorteil des offenzelligen Schaumstoffs auf Basis eines quervernetzten Polyorganosiloxans als Bestandteil der erfindungsgemäßen Vorrichtung besteht darin, dass eine Unterdruckbehandlung von Wunden ermöglicht wird, die beim Patienten einen Wechsel der Wundauflagen in Intervallen von drei Tagen oder länger ermöglicht. Während des Intervalls von drei Tagen oder länger wurde die erfindungsgemäße Wundauflage vom Patienten als angenehm empfunden. Ruckreiz und Hautrötungen wurden in der Regel vermieden. Beim Wechsel der Wundauflage nach einem Zeitraum von 3 Tagen oder mehr traten weniger unangenehme Gerüche auf als bei herkömmlichen Wundauflagen. Die Keimdichte in der ausgewechselten Wundauflage war unerwartet niedrig.

Die Bestandteile (a) bis (c) der erfindungsgemäßen Vorrichtung werden nachstehend beschrieben.

Die erfindungsgemäße Vorrichtung umfasst ein Abdeckmaterial (a) zum luftdichten Verschließen der Wunde. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass unter Berücksichtigung der eingesetzten Unterdruckpumpe der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das luftdichte Abdeckmaterial kann beispielsweise in Form einer aus einem festen Material bestehenden Schale oder in Form einer flexiblen Folie vorliegen. Vorstellbar sind auch Kombinationen von festen Rahmen oder Auflageplatten mit flexiblen Folien.

In einer bevorzugten Ausführungsform der Erfindung umfasst das Abdeckmaterial zum luftdichten Verschließen der Wunde ein wasserunlösliches Polymer, oder eine Metallfolie. Das Abdeckmaterial weist bevorzugt eine Dicke von 10 µm bis 10.000 µm, insbesondere von 25 µm bis 100 µm auf.

In einer bevorzugten Ausführungsform der Erfindung handelt es sich bei dem Abdeckmaterial (a) um ein wasserunlösliches Polymer. Bevorzugt weist das wasserunlösliche Polymer eine Löslichkeit von 10 mg/l oder weniger, mehr bevorzugt von 1 mg/ml oder weniger, insbesondere von 0,0001 bis 1 mg/ml auf (bestimmt gemäß Säulenelutionsmethode nach EU-Richtlinie RL67-548-EWG, Anhang V Kap. A6). Beispiele sind Polyurethan, Polyester, Polypropylen, Polyethylen, Polyamid oder Polyvinylchlorid, Polyorganosiloxan (Silikon), oder eine Mischung daraus. Die genannten Polymere liegen hierbei bevorzugt in nicht zellulärer Form vor.

Es hat sich gezeigt, dass durch ein Abdeckmaterial mit einer speziellen Wasserdampfdurchlässigkeit eingangs erläuterte Aufgaben besonders vorteilhaft gelöst werden können. In einer bevorzugten Ausführungsform weist daher das Abdeckungsmaterial einen Wasserdampfdurchlässigkeit von 100 bis 2500 g/m² x 24h, mehr bevorzugt von 500 bis 2000 g/m² x 24h, noch mehr bevorzugt von 800 bis 1600 g/m² x 24h, insbesondere von 1050 bis 1450 g/m² x 24h, bestimmt gemäß DIN EN 13726-2 bei 23 °C und 85 % relativer Luftfeuchte, auf. Insbesondere die Kombination einer Abdeckfolie mit vorstehend genannter Wasserdampfdurchlässigkeit mit einem offenzelligen Schaumstoff (c) mit nachstehend beschriebenen physikalischen Eigenschaften ist besonders vorteilhaft.

Als Abdeckmaterial können auch Fertigprodukte eingesetzt werden, welche die vorstehend genannten Eigenschaften aufweisen. Als besonders geeignetes Abdeckmaterial für die erfindungsgemäße Vorrichtung hat sich der Polyurethanfilm der Marke Hydrofilm^{®} (Paul Hartmann AG, Deutschland) oder Visulin^{®} (Paul Hartmann AG, Deutschland) erwiesen.

Das Abdeckmaterial wird üblicherweise in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

In einer bevorzugten Ausführungsform der Erfindung umfasst das Abdeckmaterial eine Folie aus einem oder mehreren wasserunlöslichen Polymeren, wobei die Folie vollflächig selbstklebend ausgestattet ist oder einen selbstklebenden Rand aufweist.

Möglich ist jedoch auch, dass das Abdeckmaterial lediglich durch den während der Unterdruckbehandlung erzeugten Unterdruck an der Wunde gehalten wird.

In einer bevorzugten Ausführungsform werden Abdeckmaterial und das Mittel zur funktionellen Verbindung des Wundraums mit einer außerhalb des Abdeckmaterials befindlichen Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Die erfindungsgemäße Vorrichtung zur Unterdrucktherapie umfasst ein Mittel (b) zur Herstellung von Unterdruck im Wundraum. In einer bevorzugten Ausführungsform handelt es sich hierbei um ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und die Wunde gebildete Zwischenraum verstanden. "Unterdruck" wird häufig auch als "negativer Druck" bezeichnet.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d. h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von höchstens 500 mg Hg, bevorzugt höchstens 250 mm Hg. Dieser Unterdruckbereich von höchstens 500 mg Hg, bevorzugt höchstens 250 mm Hg hat sich als für die Wundheilung geeignet erwiesen. In einer bevorzugten Ausführungsform der Erfindung handelt es sich bei dem Unterdruck um einen Unterdruck von mindestens 10 mm Hg und höchstens 150 mm Hg, mehr bevorzugt von mindestens 65 und höchstens 135 mm Hg.

Im Allgemeinen kann es sich bei dem Unterdruck um einen konstanten Unterdruck (bα) oder um einen zeitlich variierenden Unterdruck (bβ) handeln.

Unter "konstantem Unterdruck" (bα) wird hier verstanden, dass der Unterdruck während der Behandlung im Wesentlichen konstant gehalten wird. "Im Wesentlichen konstant" bedeutet, dass während der Behandlung geringfügige Veränderungen des Unterdrucks, beispielsweise um 15 % nach oben oder unten, auftreten können.

Ein bevorzugter konstanter Unterdruck ist der Bereich von mindestens 80 mm Hg bis höchstens 150 mm Hg.

Unter "zeitlich variierendem Unterdruck" (bβ) wird hier verstanden, dass der Unterdruck während der Behandlung gezielt variiert wird. Unter der gezielten Variation des Luftdrucks werden diejenigen Variationen des Luftdrucks verstanden, die einsetzen, wenn nach Anlegen des Unterdruckverbandes ein erster Sollwert für den Unterdruck erreicht wurde. Dagegen ist die erste Anstiegsphase des Unterdrucks, die nach dem Anlegen des Verbandes bis zum Erreichen des ersten Sollwertes auftritt, nicht von dem Begriff "zeitlich variierender Unterdruck" umfasst. Dies gilt analog für die am Ende der Behandlung nötige Absenkung des Luftdrucks auf den Umgebungsluftdruck, die gleichfalls nicht von dem Begriff "zeitlich variierender Unterdruck" umfasst ist.

Der "zeitlich variierende Unterdruck" ist durch den Umgebungsluftdruck nach unten begrenzt und durch einen maximalen Unterdruck von 500 mm Hg, bevorzugt 250 mm Hg, mehr bevorzugt 150 mm Hg, insbesondere 125 mm Hg nach oben begrenzt. Der während der Behandlung applizierte tatsächliche Unterdruck bewegt sich innerhalb dieser durch ihre Eckwerte definierten Spanne. Der "zeitlich variierende Unterdruck" umfasst also beispielsweise einen ein- oder mehrmaligen Wechsel zwischen einem oder mehreren höheren Unterdruckwerten und einem oder mehreren geringeren Unterdruckwerten. Ebenso umfasst der "zeitlich variierende Unterdruck" einen während der Behandlung erfolgenden gezielten ein- oder mehrmaligen Wechsel zwischen dem Umgebungsdruck und einem oder mehreren höheren Unterdruckwerten.

Bei einer bevorzugten Ausführungsform beträgt der maximale Unterdruck für einen zeitlich variierenden Unterdruck 125 mm Hg. Die untere Grenze für die Variation des Unterdrucks stellt bei dieser Ausführungsform der Umgebungsluftdruck dar. Während der Behandlung variiert der Unterdruck zwischen oder innerhalb dieser Eckwerte.

Bei einer weiteren bevorzugten Ausführungsform beträgt der maximale Unterdruck für einen zeitlich variierenden Unterdruck 125 mm Hg. Die untere Grenze für die Variation des Unterdrucks beträgt bei dieser Ausführungsform 20 mm Hg. Während der Behandlung variiert der Unterdruck zwischen oder innerhalb dieser Eckwerte.

Bei beiden vorgenannten Ausführungsformen kann der Wechsel zwischen dem oberen und dem unterem Druckwert periodisch oder nicht-periodisch erfolgen. Ein periodischer Wechsel ist bevorzugt. Die Zeitintervalle, in denen der höhere Unterdruck und in denen der geringere Unterdruck oder Umgebungsdruck gehalten wird, können jeweils unterschiedlich lang sein. Vorzugsweise wird ein geringerer Unterdruck länger gehalten als ein höherer Unterdruck. Geeignete Zeitintervalle, in denen jeweils eine bestimmte Unterdruckeinstellung oder der Umgebungsdruck gehalten wird, sind beispielsweise 1 min, 2 min, 5 min, 10 min, 30 min, 1 h, 12 h oder 24 h.

Besonders bevorzugt ist ein variierender Unterdruck mit den nachstehend genannten Parametern, wobei während der Behandlung kontinuierlich in den angegebenen Zeitabständen zwischen den beiden Unterdruckwerten gewechselt wird:
Ein Unterdruck von 125 mm Hg während 2 min,
danach
ein Unterdruck von 20 mm Hg während 5 min.

Die erfindungsgemäße Vorrichtung kann weiterhin ein Mittel umfassen, so dass der in der Vorrichtung tatsächlich vorhandene Unterdruck überprüfbar und gegebenenfalls einstellbar ist. Das Mittel kann sich im Wundraum oder an einer anderen geeigneten Stelle befinden. So ist es beispielsweise möglich, einen Drucksensor in der Unterdruckleitung zwischen Wundverband und der Unterdruckquelle anzubringen.

Die erfindungsgemäße Vorrichtung zur Unterdrucktherapie von Wunden umfasst weiterhin ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel zur funktionellen Verbindung des Wundraums mit einer außerhalb des Abdeckmaterials befindlichen Unterdruckquelle um mindestens eine Verbindungsleitung. Die mindestens eine Verbindungsleitung kann durch das Abdeckmaterial hindurchgeführt werden.

Alternativ kann die mindestens eine Verbindungsleitung unter dem Rand des Abdeckmaterials durchgeführt werden.

In beiden Fällen ist die Durchtrittsstelle luftdicht abzudichten, so dass der gewünschte Unterdruck im Verband aufrecht erhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silicon-Drainageschlauch.

Die Verbindungsleitung verfügt zweckmäßigerweise an dem Ende, welches sich außerhalb des Wundverbandes befindet, über einen Unterdruckadapter, um mit den weiteren Komponenten des Unterdrucksystems verbindbar zu sein.

Die Verbindungsleitung verfügt an dem Ende, welches sich innerhalb des Wundverbandes befindet, eine Öffnung.

In einer weiteren Ausführungsform handelt es sich bei dem Mittel zur funktionellen Verbindung des Wundraums mit einer außerhalb des Abdeckmaterials befindlichen Unterdruckquelle um ein Unterdruck-Anschlussstück (Port), welches auf einer der Innen- oder Außenseiten des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden. Es ist auch denkbar, dass der Port selbst über entsprechende Befestigungseinrichtungen, wie beispielsweise Klebeflächen, verfügt.

Geeignete Unterdruck-Anschlussstücke sind kommerziell erhältlich. Dabei handelt es sich typischerweise um Unterdruck-Anschlussstücke, welche auf der Außenseite des Abdeckmaterials befestigt werden.

Auch das Unterdruck-Anschlussstück verfügt zweckmäßigerweise über einen Unterdruckadapter, um mit den weiteren Komponenten des Unterdrucksystems verbindbar zu sein.

Neben den vorstehend beschriebenen Bestandteilen (a) und (b) umfasst die erfindungsgemäße Vorrichtung noch Bestandteil (c). Der offenzellige Schaumstoff auf Basis eines quervernetzten Polyorganosiloxans (c), welcher in der erfindungsgemäßen Vorrichtung Verwendung findet, wird nachstehend genauer beschrieben.

Als Schaumstoffe werden üblicherweise Werkstoffe mit über die gesamte Masse verteilten Zellen (offen, geschlossen oder beides) verstanden. Werkstoffe weisen üblicherweise somit eine Rohdichte (gemäß DIN 53420) auf, die niedriger ist als die Dichte der Gerüstsubstanz.

Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff (c) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.

Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke des übrigen Bereichs der Zellwand auf.

Unter einem retikulierten Schaumstoff wird ein Schaumstoff verstanden, der im Wesentlichen nur Zellstege aufweist.

Allgemein werden unter Polysiloxanen synthetische Polymere verstanden, bei denen Siliciumatome über Sauerstoffatome verknüpft sind.

Unter "quervernetzt" wird üblicherweise verstanden, dass lineare Polymerketten über kovalente Bindungen quer (d.h. nicht am Ende der linearen Ketten) miteinander verbunden sind. In einer bevorzugten Ausführungsform kommt die Quervernetzung (zumindest teilweise) dadurch zustande, dass lineare Polysiloxanketten über Kohlenstoffatome kovalent verbunden werden. Bevorzugt ist diese Quervernetzung über Kohlenstoffatome erhältlich durch Umsetzung eines Polyorganosiloxan, enthaltend eine oder mehrere Gruppen mit einer C₂-C₆-Alkenylgruppe mit einem Polyorganosiloxan, enthaltend eine oder mehrere Si-H Gruppen.

In einer bevorzugten Ausführungsform ist der Schaumstoff (c) erhältlich durch Umsetzung einer härtbaren Mischung umfassend die Komponenten:
(i) ein Polyorganosiloxan enthaltend eine oder mehrere Gruppen mit einer C₂-C₆-Alkenylgruppe, bevorzugt enthaltend eine oder mehrere Vinyl-Gruppen,
(ii) ein Polyorganosiloxan enthaltend eine oder mehrere Si-H-Gruppen,
(iii) ein Treibmittel enthaltend eine oder mehrere OH-Gruppen, und
(iv) einen metallorganischen Katalysator.

Die Komponenten (i) bis (iv) der härtbaren Mischung werden nachstehend näher erläutert.

Bei der Komponente (i) kann es sich Polyorganosiloxan handeln, das sogenannte "M, D, T und Q" -Einheiten aufweist. Hierbei handelt es sich um die im Stand der Technik anerkannte Konvention zur Bezeichnung von strukturellen Polysiloxan-Einheiten in Übereinstimmung mit der Anzahl der an Silicium gebundenen Sauerstoffatome. Diese Konvention benutzt die Buchstaben M, D, T und Q, um die Anzahl der Sauerstoffatome als Abkürzungen für "mono", "di", "tri" bzw. "tetra" zu benutzen.

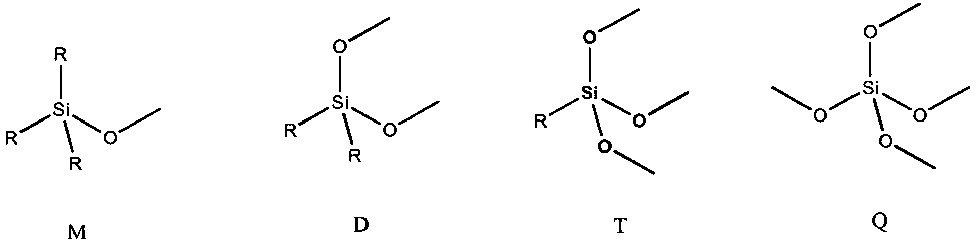

In einer bevorzugten Ausführungsform enthält die Komponente (i) der härtbaren Mischung ein Polyorganosiloxan gemäß der allgemeinen Formel (1): 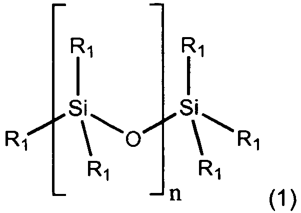 in der R¹ unabhängig C₁₋₆-Alkyl, C₁₋₆-Haloalky, Aryl oder C₂-C₆-Alkenyl ist, mit der Maßgabe, dass das Molekül mindestens eine C₂-C₆-Alkenylgruppe, bevorzugt mindestens zwei C₂-C₆-Alkenylgruppen oder mindestens drei C₂-C₆-Alkenylgruppen enthält. Bei der Verbindung gemäß Formel (1) handelt es sich somit bevorzugt um ein lineares Polyorganosiloxan, das M- und D-Einheiten aufweist.

Bei den C₂-C₆-Alkenylgruppen kann es sich beispielsweise um Vinyl-, Allyl- und/oder Hexenylgruppen handeln. Bevorzugt handelt es sich bei der C₂-C₆-Alkenylgruppe um eine Vinylgruppe. Es ist ferner bevorzugt, dass die C₂-C₆-Alkenylgruppe, insbesondere die Vinylgruppe, an den terminalen Si-Atomen angebracht ist, wie in Formel (1a) veranschaulicht wird, 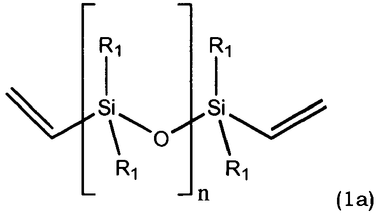 wobei R¹ unabhängig C₁₋₆-Alkyl, C₁₋₆-Haloalky, Aryl oder C₂-C₆-Alkenyl ist. Insbesondere ist in Formel (1a) R¹ = Methyl.

Das Polyorganosiloxan gemäß Formel (1) bzw. (1a) wird im Rahmen dieser Anmeldung auch als Bestandteil (i-M/D) der härtbaren Mischung bezeichnet. Üblicherweise wird "n" so gewählt, dass die Viskosität des Polyorganosiloxans im Bereich von 500-250.000 mPas, bevorzugt von 600 bis 100.000 mPas, mehr bevorzugt von 1000 bis 50.000 mPas, noch mehr bevorzugt von 1.500 bis 20.000 mPas, insbesondere von 2.000 bis 10.000 mPas liegt. Die Viskosität wird im Rahmen dieser Anmeldung grundsätzlich bei 23 °C gemessen und gemäß Ph. Eur. 1997, Methode 2.2.10 (Rotationsviskosimeter) mit Hilfe eines Brookfield Viskosimeters Modell DV-II (Brookfield Engineering Laboratories Vertriebs GmbH, D-73547 Lorch, Deutschland) bestimmt.

Neben dem linearen Bestandteil (i-M/D) kann die Komponente (i) der härtbaren Mischung (d.h. das Polyorganosiloxan enthaltend eine oder mehrere Gruppen mit einer C₂-C₆-Alkenylgruppe) auch verzweigte Polysiloxan-Bestandteile enthalten, die neben M- und D-Einheiten auch T- und/oder Q-Einheiten aufweisen können. Diese verzweigten Bestandteile werden als Bestandteil (i-M/D/T/Q) bezeichnet. Auch auf den Bestanteil (i-M/D/T/Q) finden die vorstehend angegebenen Erläuterungen zu Art und Menge der C₂-C₆-Alkenylgruppen Anwendung. Insbesondere enthält auch der Bestandteil (i-M/D/T/Q) mindestens zwei C₂-C₆-Alkenylgruppen, bevorzugt Vinylgruppen.

Verzweigungsgrad und Kettenlänge werden beim Bestandteil (i-M/D/T/Q) in der Regel so gewählt, dass die Viskosität des Polyorganosiloxans im Bereich von 500-250.000 mPas, bevorzugt von 600 bis 100.000 mPas, mehr bevorzugt von 1000 bis 50.000 mPas, noch mehr bevorzugt von 1.200 bis 20.000 mPas, insbesondere von 1.500 bis 10.000 mPas liegt.

Bevorzugt ist in Komponente (i) der härtbaren Mischung das Gewichtsverhältnis von Bestanteil (i-M/D) zu (i-M/D/T/Q) von 20 : 1 bis 1 : 10, mehr bevorzugt von 10 : 1 bis 1 : 2, noch mehr bevorzugt von 8 : 1 bis 1 : 1, besonders bevorzugt von 6 : 1 bis 1 : 1,5, insbesondere von 5 : 1 bis 1 : 2.

Als Komponente (ii) umfasst die härtbare Mischung ein Polyorganosiloxan enthaltend eine oder mehrere Si-H-Gruppen. Bevorzugt enthält dieses Polyorganosiloxan mindestens zwei, insbesondere mindestens drei Si-H-Gruppen pro Molekül. Es ist ferner bevorzugt, dass pro Si-Atom nicht mehr als eine Si-H-Bindung vorliegt.

Üblicherweise ist in der Komponente (ii) an 0,01 bis 10 %, bevorzugt 0,05 bis 5 % der Siliciumatome eine C₂-C₆-Alkenylgruppe gebunden.

In einer bevorzugten Ausführungsform enthält die Komponente (ii) ein Polyorganosiloxan gemäß allgemeiner Formel (2), 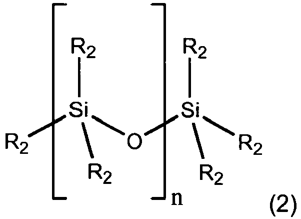 in der R² unabhängig C₁₋₆-Alkyl, C₁₋₆-Haloalky, Aryl oder Wasserstoff ist. Bevorzugt gilt dies mit der Maßgabe, dass ein Molekül gemäß Formel (2) mindestens zwei, bevorzugt mindestens 3 an Silicium gebundene Wasserstoffatome enthält, wobei diese an unterschiedliche Siliciumatome gebunden sind. In vorstehender Formel (2) weist das Polyorganosiloxan nur M- und D-Einheiten auf, was bevorzugt ist. Alternativ kann das Polyorganosiloxan auch zusätzlich T- und/oder Q-Einheiten aufweisen. Üblicherweise weisen in der Komponente (ii) 0,01 bis 10 % der Siliciumatome eine Si-H-Bindung auf.

Verzweigungsgrad und Kettenlänge werden beim Bestandteil (ii) in der Regel so gewählt, dass die Viskosität des Polyorganosiloxans im Bereich von 500 - 150.000 mPas, bevorzugt von 600 bis 50.000 mPas, mehr bevorzugt von 1.000 bis 20.000 mPas, noch mehr bevorzugt von 1.200 bis 15.000 mPas, insbesondere von 1.500 bis 10.000 mPas, liegt.

In einer bevorzugten Ausführungsform enthält das Polysiloxan (ii) M- und D-Einheiten, wobei lediglich die D-Einheiten Si-H-Verbindungen umfassen. Die M-Einheiten enthalten hierbei bevorzugt Methylreste. Ein entsprechendes Polyorganosiloxan ist in Formel (2a) veranschaulicht 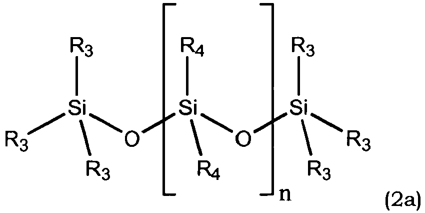 in der R³ ein Aryl- oder Alkylrest, insbesondere Methyl, und R⁴ ein Aryl- oder Alkylrest oder Wasserstoff, insbesondere Methyl oder Wasserstoff, ist.

Durch Umsetzung der Komponente (i) mit der Komponente (ii) der härtbaren Mischung entsteht ein quervernetztes Polyorganosiloxan.

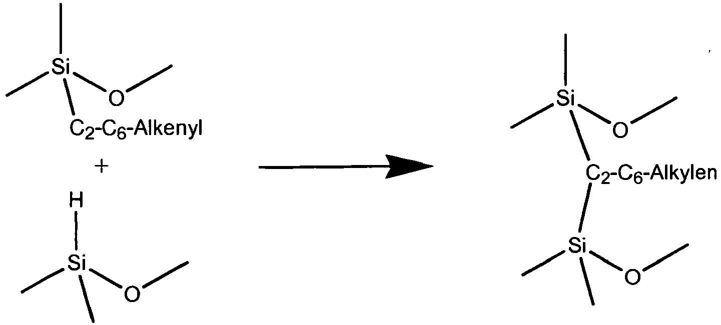

Neben den Bestandteilen (i) und (ii) enthält die härtbare Mischung des Weiteren noch ein Treibmittel (iii), bevorzugt ein Treibmittel enthaltend eine oder mehrere OH-Gruppen. Beispiele für Verbindungen, die eine oder mehrere OH-Gruppen enthalten, sind Wasser (H-OH), Polyorganosiloxane mit Si-OH-Gruppen und Alkohole. Bevorzugt werden als Treibmitttel (iii) C₁-C₁₂-Akohole verwendet. Insbesondere besteht Komponente (iii) aus einem oder mehreren C₁-C₁₂-Akoholen. Im Allgemeinen sind Monoalkohole oder Di- oder Polyalkohole möglich. Bevorzugt werden Monoalkohole eingesetzt. Beispiele für geeignete Alkohole sind Monoalkohole wie Methanol, Ethanol, 1-Propanol, 2-Propanol, 1-Butanol, 2-Butanol, t-Butanol, 1-Pentanol, cyclo-Pentanol, 1-Hexanol, 2-Hexanol, cyclo-Hexanol, Heptanol, Octanol oder auch Dialkohole wie Ethylenglykol, Propylenglykol, 1,4-Butandiol.

Besonders bevorzugt werden Monoalkohole mit 4 bis 6 Kohlenstoffatomen verwendet, insbesondere 1-Butanol, 1-Pentanol und 1-Hexanol.

Es hat sich zu Erreichung der nachstehend beschriebenen physikalischen Eigenschaften gezeigt, dass es vorteilhaft ist, wenn die Wassermenge in der härtbaren Mischung begrenzt ist. Bevorzugt weist die härtbare Mischung einen Wassergehalt von 0,0001 bis 3 Gew.-%, mehr bevorzugt von 0,001 bis 2,0 Gew.-%, noch mehr bevorzugt von 0,01 Gew.-% bis 1,0 Gew.-%, auf. Der Wassergehalt wird hierbei nach der coulometrischen Karl-Fischer-Methode bestimmt. Üblicherweise ergibt sich der Wassergehalt nicht durch Zugabe von Wasser, sondern durch den Restwassergehalt der Komponenten (i) bis (iv) der härtbaren Mischung.

Durch Umsetzung der Komponente (iii) mit der Komponente (ii) erfolgt eine sogenannte Treibreaktion. Dies führt zu Herstellung von zellulären Polyorganosiloxanen.

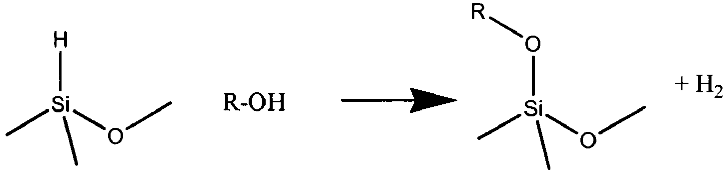

Neben den Komponenten (i) bis (iii) enthält die härtbare Zusammensetzung einen metallorganischen Katalysator (iv). Der metallorganische Katalysator katalysiert üblicherweise die Reaktion der Komponente (i) mit der Komponente (ii) und/oder die Reaktion mit der Komponente (ii) mit der Komponente (iii).

Bevorzugt handelt es sich um metallorganische Verbindungen, wobei das Metall aus der Gruppe 8 (Eisengruppe), Gruppe 9 (Cobaltgruppe), Gruppe 10 (Nickelgruppe) oder Gruppe 11 (Kupfergruppe) ausgewählt ist. Mehr bevorzugt handelt es sich bei dem Metall um Palladium oder Platin, insbesondere Platin.

Es sind zahlreiche Arten von Platin-Katalysatoren im Stand der Technik bekannt. Sie schließen z. B. Reaktionsprodukte von Chlorplatinsäure mit Olefinen, Alkoholen, Ethern, Aldehyden und Vinylsiloxanen, wie Tetramethyldivinyldisiloxan, ein. Ein Reaktionsprodukt von Chlorplatinsäure mit Tetramethyldivinyldisiloxan in Gegenwart von Natriumbicarbonat, wie in der US-PS 3,775,452 offenbart, gelöst in Xylol bis zu einer Menge von etwa von 5 Gew.-% Platin, ist bevorzugt und wird im folgenden als "Karstedt-Katalysator" bezeichnet.

Weitere Beispiele für geeignete Platinkatalysatoren sind:
Pt(CH₃CN)₂Cl₂,
{Pt(CH₂CN)₂(CH₃)₄}Cl₂,
Pt(NH₃)₂Cl₂,
K{PtCl₃CH₂CH₂CH₂ OH)},
PtBr₂(CH₂H₄)₂,
K{PtBr₃(C₂H₄)},
PtCl₂(C₂H₄),
(CH₃)₂C=CH₂PtCl₂,
H₂Pt(CN)₄ • 5H₂O,
H{PtCl₃(CH₂CN)},
Pt(NH₃)₂(CNS)₂,
PtCl₂ PCl₃,
{Pt(NH₃)₄} {PTCl₄},
PtCl₂ {P(CH₂CH₃)₃}₂,
PtCl₂ {P(OCH₂CH₃)₃}₂,
Pt(OOCH₂SCH₂CH₃)₂,
(CH₃)₄Pt,
(CH₃)₃Pt - Pt(CH₃)₃,
PtCl₂CO
und
PtBr₂CO.

In der härtbaren Mischung werden im Allgemeinen Mengen von 100 Gewichtsteilen Komponente (i);
1 bis 150 Gewichtsteile, bevorzugt 10 bis 80 Gewichtsteile, mehr bevorzugt 15 bis 50 Gewichtsteile Komponente (ii);
1 bis 100, bevorzugt 2 bis 50, mehr bevorzugt 5 bis 30 Gewichtsteile Komponente (iii); und
0,0001 bis 10 Gewichtsteile, bevorzugt 0,001 bis 5 Gew.-%, mehr bevorzugt 0,01 bis 3 Gewichtsteile Komponente (iv) bevorzugt.

Das molare Verhältnis von Si-H-Gruppen in der Komponente (ii) zur Summe aus C₂-C₅-Alkenylgruppen aus der Komponente (i) und OH-Gruppen aus der Komponente (iii) beträgt üblicherweise 5 : 1 bis 1 : 10, bevorzugt 4 : 1 bis 1 : 5, mehr bevorzugt 3 : 1 bis 1 : 4, noch mehr bevorzugt 2 : 1 bis 1 : 3, besonders bevorzugt 1,5 : 1 bis 1 : 2 und insbesondere 1 : 0,9 bis 1 : 1,5.

Neben den Komponenten (i) bis (iv) kann die härtbare Mischung ferner noch weitere, im Fachgebiet übliche Zusatzstoffe, wie Füllstoffe und Additive, enthalten.

Um einen offenzelligen Silikonschaumstoff (c) herzustellen, werden die oben beschriebenen Komponenten in irgendeiner erwünschten Reihenfolge vermengt. Es ist bevorzugt, dass eine erste Teilmischung A, enthaltend die Komponenten (i), (iii) und (iv), mit einer zweiten Teilmischung B, enthaltend die Komponenten (i), (ii) und (iii), vermischt wird. Die Vermischung kann durch übliche Mischgeräte erfolgen, beispielsweise mit einem statischem Mischer oder einem dynamischen Mischer.

Die Umsetzung der Komponenten (i) bis (iv) der härtbaren Zusammensetzung erfolgt bevorzugt bei 20 bis 25 °C, insbesondere bei 23 °C.

Die vorstehend erläuterten Komponenten (i) bis (iv) werden bevorzugt so gewählt, dass ein offenzelliger Schaumstoff auf Basis eines quervernetzten Polysiloxans, insbesondere ein Schaumstoff mit den nachfolgend beschriebenen physikalischen Eigenschaften erhalten wird.

Es hat sich gezeigt, dass vorstehend genannte Aufgaben unerwartet vorteilhaft gelöst werden können, wenn der Schaumstoff (c) eine spezielle Zugfestigkeit, eine spezielle Bruchdehnung und einer spezielle Härte aufweist. In einer bevorzugten Ausführungsform weist der Schaumstoff (c) eine Zugfestigkeit von 100 kPa bis 100 MPa, mehr bevorzugt 500 kPa bis 50 MPa, noch mehr bevorzugt 800 kPa bis 10 MPa, gemessen gemäß DIN 53571, auf. Ferner weist der Schaumstoff (c) bevorzugt eine Bruchdehnung von 100 % bis 350 %, mehr bevorzugt von 160 % bis 320 %, noch mehr bevorzugt von 180 % bis 300 %, gemessen gemäß DIN 53571, auf. Zudem weist der Schaumstoff (c) bevorzugt eine Härte von 20 bis 70 Shore A, mehr bevorzugt von 30 bis 60 Shore A, noch mehr bevorzugt von 40 bis 50 Shore A, gemessen gemäß DIN 53505, auf.

Es hat sich ferner gezeigt, dass vorstehend genannte Aufgaben unerwartet vorteilhaft gelöst werden können, wenn der Schaumstoff (c) eine spezielle Luftdurchlässigkeit aufweist. In einer bevorzugten Ausführungsform weist der Schaumstoff (c) eine Luftdurchlässigkeit von 1000 bis 8000 1/(m²sec), mehr bevorzugt von 2000 bis 7500 1/(m²sec), noch mehr bevorzugt von 2500 bis 7000 1/(m²sec), insbesondere von 3000 bis 6500 1/(m²sec), gemessen gemäß DIN EN ISO 9237, auf.

Es hat sich weiterhin gezeigt, dass vorstehend genannte Aufgaben unerwartet vorteilhaft gelöst werden können, wenn der Schaumstoff (c) viskoelastisches Verhalten zeigt. Hierunter wird verstanden, dass das Verhalten des Schaumstoffs (c) auf Beanspruchung so aussieht, wie die Kombination aus einem elastischen Feststoff und einer viskosen Flüssigkeit. Das viskoelastische Verhalten kann durch einen Torsionsschwingungsversuch gemäß DIN 53445 charakterisiert werden. Es ist bevorzugt, dass der Schaumstoff bei der Bestimmung gemäß DIN 53445 bei 23 °C einen mechanischen Verlustfaktor von 0,1 bis 1,0, mehr bevorzugt von 0,15 bis 0,8, noch mehr bevorzugt von 0,2 bis 0,6, aufweist.

Es hat sich weiterhin gezeigt, dass vorstehend genannte Aufgaben unerwartet vorteilhaft gelöst werden können, wenn der Schaumstoff (c) eine Rohdichte zwischen 0,12 und 0,35 kg/m³, mehr bevorzugt wischen 0,15 und 0,25 m³, insbesondere zwischen 0,18 und 0,22 m³ aufweist, gemessen gemäß DIN EN ISO 845.

Es hat sich weiterhin gezeigt, dass vorstehend genannte Aufgaben unerwartet vorteilhaft gelöst werden können, wenn der Schaumstoff (c) Silber in Form von Silberionen oder in Form von atomarem Silber enthält. Bevorzugt wird nach der Herstellung des Schaumstoffs (c) eine Silberbeschichtung aufgebracht. Alternativ kann das Silber bereits in die härtbare Zusammensetzung mit eingebracht werden. Bevorzugt enthält der Schaumstoff (c) 0,000001 bis 0,1 Gew.-%, mehr bevorzugt 0,0001 bis 0,01 Gew.-% Silber, bezogen auf das Gesamtgewicht des Schaumstoffs (c).

In einer bevorzugten Ausführungsform wird der offenzellige Schaumstoff in trockenem Zustand eingesetzt. Bevorzugt ist somit der Schaumstoff z.B. nicht mit einer Aktivierungslösung (wie z.B. Ringerlösung) getränkt.

In einer bevorzugten Ausführungsform der Erfindung weist der offenzellige Schaumstoff eine Dicke von 1 bis 50 mm, insbesondere von 15 mm bis 30 mm auf.

In einer bevorzugten Ausführungsform der Erfindung umfasst die Vorrichtung zur Unterdrucktherapie von Wunden mindestens eine Wundkontaktschicht zum Einbringen zwischen Schaumstoff (c) und Wundoberfläche. Die zusätzliche Wundkontaktschicht kann mit dem Schaumstoff (c) haftend oder nicht haftend verbunden sein.

Als Wundkontaktschicht kommt grundsätzlich jede aus dem Stand der Technik bekannte Wundkontaktschicht in Frage, solange einerseits ein Durchtritt von Wundexsudat gewährleistet ist und das Material anderseits keine Neigung zum Verwachsen oder Verkleben mit dem Wundgewebe aufweist.

Beispiele für geeignete Wundkontaktschichten sind in den Deutschen Patentanmeldungen DE 10 2008 062 472.1, DE 10 2008 031 183.9 und DE 10 2008 031 182.0 beschrieben.

Ebenso kann die Wundkontaktschicht eine durchlässige Non-woven-Schicht oder einen Gittertüll umfassen. Die durchlässige Non-woven-Schicht oder der Tüll besteht vorzugsweise aus einem hydrophoben Material, beispielsweise Polyester. Der Tüll kann weiterhin mit einer Salbe und/oder einer Silberbeschichtung ausgestattet sein. Besonders geeignete Wundkontaktschichten sind Salbenkompressen der Marke Hydrotüll^{®} und Atrauman^{®} (Paul Hartmann AG, Deutschland).

In einer weiteren bevorzugten Ausführungsform umfasst die Vorrichtung zur Unterdrucktherapie von Wunden mindestens eine zusätzliche Druckverteilungsschicht zwischen dem Schaumstoff (c) und dem Abdeckmaterial (a).

Der Vorteil einer zusätzlichen Druckverteilungsschicht kann darin bestehen, dass der durch den Verband auf den Wundgrund ausgeübte Druck durch die Verwendung der Druckverteilungsschicht noch gleichmäßiger verteilbar ist. Weiterhin kann die Druckverteilungsschicht zusätzliches Wundexsudat speichern.

Die zusätzliche Druckverteilungsschicht kann aus einem offenzelligen oder halboffenzelligen Schaumstoff, einem Abstandsgewirke, aus einer Textilschicht, aus einem strukturiertem Gel, oder aus einer durchlässigen Non-woven-Schicht bestehen. Geeignete Textilschichten sind unter anderem ES-Kompressen oder Gittertülls.

Die zusätzliche Druckverteilungsschicht kann so ausgebildet sein, dass Flüssigkeit, wie Wundexsudat, durch sie hindurchgeleitet wird. Dazu kann die Druckverteilungsschicht geeignete Kanäle oder Öffnungen enthalten, oder aus einem für Flüssigkeiten durchlässigen Material bestehen.

Weiterhin stellt die Erfindung ein gebrauchsfertiges Set zur Unterdruck-Wundbehandlung, umfassend die erfindungsgemäße Vorrichtung, wobei der Schaumstoff (c) als Wundauflage geeignet ist und gebrauchsfertig abgepackt vorliegt.

Der vom Set umfasste abgepackte Schaumstoff (c) kann hergestellt werden, indem der trockene Schaumstoff (c) feuchtigkeitsdicht abgepackt wird. Vorzugsweise liegt der gebrauchsfertig abgepackte Schaumstoff (c) in steriler Form vor. Idealerweise liegt der Schaumstoff (c) in steriler, einzeln eingesiegelter, gebrauchsfertiger Packung vor. Die Sterilisation kann durch eine Gegendruckdampfsterilisation oder durch andere, dem Fachmann als geeignet bekannte Sterilisationsmethoden erfolgen.

Das Set kann weitere optionale Bestandteile wie beispielsweise eine oder mehrere Wundkontaktschichten, eine oder mehrere zusätzliche Druckverteilungsschichten, Klebemittel zum Fixieren des Verbands, Abdichtmittel zur Herstellung einer luftundurchlässigen Abdichtung des Verbandes, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Bevorzugt enthält das erfindungsgemäße Set ferner Schere, Tupfer und/oder Pinzette, insbesondere in steriler Form.

Das Set kann sowohl die mindestens eine Wundkontaktschicht, als auch die mindestens eine zusätzliche Druckverteilungsschicht umfassen.

Vorzugsweise umfasst das Set weiterhin eine gebrauchsfertige Unterdruckeinheit. Die Unterdruckeinheit kann Komponenten wie beispielsweise eine Pumpe, ein oder mehrere Flüssigkeitsbehälter, eine Steuereinheit, eine Stromversorgung, elektrische Anschlussmitteln und Schläuche enthalten. Die Unterdruckeinheit kann auch eine Vorrichtung zur funktionellen Verbindung des Unterdruckverbandes mit einer vorhandenen stationären Unterdruckquelle enthalten. Vorzugsweise werden alle Komponenten, bei denen dies aus medizinischer Sicht notwendig ist, steril abgepackt zur Verfügung gestellt. Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können.

### Figuren

Nachstehend wird die erfindungsgemäße Vorrichtung zur Unterdrucktherapie von Wunden anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
- **Figur 1:**: schematischer Aufbau der erfindungsgemäßen Vorrichtung (Seitenansicht)
- **Figur 2:**: schematischer Aufbau einer weiteren Ausführungsform mit Wundkontaktschicht
- **Figur 3:**: schematischer Aufbau einer weiteren Ausführungsform mit Druckverteilungsschicht

### Figurenlegende

1 Wundumgebung (d.h. in der Regel unversehrte Haut)
2 Luftundurchlässiges Abdeckmaterial (a)
3 Offenzelliger Schaumstoff auf Basis eines quervernetzten Polysiloxans (c)
4 Unterdruck- Anschlussstück (Port)
5 Unterdruck-Verbindungsleitung
6 Sammelgefäß
7 Unterdruckeinheit
8 Wundgrund
9 Druckverteilungsschicht
10 Wundkontaktschicht

### Beschreibung der Figuren

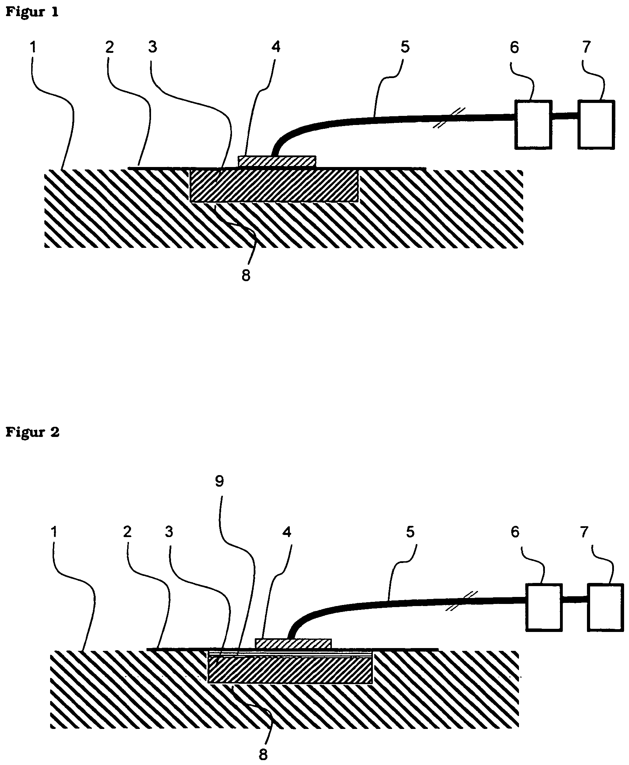

In **Figur 1** wird der schematische Aufbau der erfindungsgemäßen Vorrichtung in der Seitenansicht dargestellt. Die Vorrichtung umfasst ein luftundurchlässiges Abdeckmaterial (2), ein Mittel (4-5) zur funktionellen Verbindung des Wundraums mit einer außerhalb des Abdeckmaterials befindlichen Unterdruckquelle (7), sowie dem offenzelligen Polysiloxanschaumstoff (3). Das Abdeckmaterial (2) ist im Bereich der Wundumgebung (1), welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung (1) befestigt werden kann. Das Abdeckmaterial (2) kann in verschiedenen Abmessungen und Formen vorliegen, beispielsweise kreisförmig, oval oder rechteckig. Es kann auch in einer an die Wunde angepassten, unregelmäßigen Form vorliegen. Bei dem Abdeckmaterial (2) kann es sich um ein undurchsichtiges Material, um ein teilweise durchsichtiges Material, oder um ein vollständig durchsichtiges Material handeln. Das Abdeckmaterial (2) wird üblicherweise im Bereich der Wundumgebung (1) befestigt und luftdicht abgedichtet. Dies kann beispielsweise dadurch erfolgen, dass das Abdeckmaterial (2) einen Kleberand aufweist. Der Kleberand sollte möglichst bis zum Anlegen des Verbandes durch Schutzstreifen geschützt sein. Alternativ kann eine klebende Substanz entweder auf den Rand des Abdeckmaterials (2) und/oder auf die intakte Haut im Bereich der Wundumgebung aufgebracht werden. Dies hat den Vorteil, dass eine Anpassung des Abdeckmaterials an die Form und Größe der Wunde leichter möglich ist. Eine Befestigung und luftdichte Abdichtung der Vorrichtung kann aber auch durch die Verwendung von Klebestreifen oder einer Klebemasse erreicht werden. Das Unterdruckanschlussstück (4) ist bei der hier gezeigten bevorzugten Ausführungsform auf der von der Wunde weg weisenden Außenseite des luftundurchlässigen Abdeckmaterials (2) angebracht. Um den Wundraum mit einer außerhalb des Abdeckmaterials befindlichen Unterdruckeinheit (7) funktionell zu verbinden, muss sich bei dieser Anordnung im Bereich des Unterdruckanschlussstücks (4) eine oder mehrere durch das Abdeckmaterial (2) hindurchgehende Öffnung(en) befinden. Weiterhin ist eine luftdichte Abdichtung zu gewährleisten. Eine solche Abdichtung kann beispielsweise hergestellt werden, indem auf der von der Wunde weg weisenden Oberseite des Ports eine Folie (in Figur 1 nicht gezeigt) aufgebracht wird, die mit dem Abdeckmaterial (2) verklebt wird. Das Anlegen des Verbandes kann erleichtert werden, wenn ein Port verwendet wird, bei dem ein geeignetes Befestigungs- und Abdichtmittel zum Befestigen des Ports auf dem Abdeckmaterial bereits vorhanden ist. Dies ist beispielsweise bei dem im Handel erhältlichen PPM-Drainageport^{®} der Firma Phametra-Pharma und Medica-Trading GmbH (Herne/Ruhrstadt, Deutschland) der Fall.

**Figur 1** zeigt weiterhin einen offenzelligen Polysiloxanschaumstoff (3). Beim Anlegen des Verbandes sollte dieser so in den Wundraum eingelegt werden, dass möglichst wenige Hohlräume entstehen und ein guter Kontakt mit dem Wundgrund (8) gewährleistet ist

Der Einsatz einer Wundkontaktschicht (10) in der erfindungsgemäßen Vorrichtung wird in **Figur 2** exemplarisch in der Seitenansicht dargestellt. Hierbei handelt sich um eine bevorzugte Ausführungsform der erfindungsgemäßen Vorrichtung. Die Wundkontaktschicht (10) sollte so beschaffen sein, dass das Wundexsudat die Wundkontaktschicht (10) passieren kann. Es ist auch möglich mehrere Wundkontaktschichten zu verwenden. Dabei können auch unterschiedliche Wundkontaktschichten miteinander kombiniert werden.

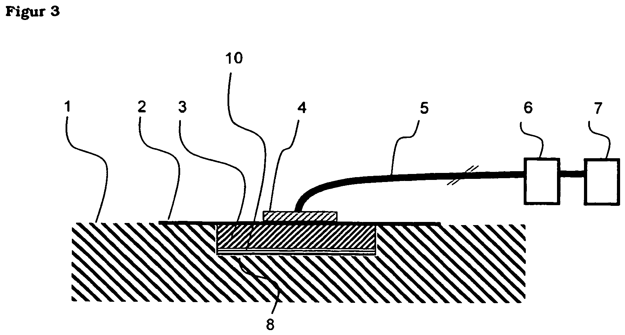

**Figur 3** zeigt den schematischen Aufbau einer weiteren bevorzugten Ausführungsform der erfindungsgemäßen Vorrichtung in der Seitenansicht. Diese Ausführungsform zeichnet sich durch die Anwesenheit einer zusätzlichen Druckverteilungsschicht (9) aus. Es ist auch möglich mehrere Druckverteilungsschichten zu verwenden, wobei unterschiedliche Druckverteilungsschichten miteinander kombiniert werden können. Hervorzuheben ist, dass auch eine Kombination der in Figur 2 gezeigten Wundkontaktschicht mit der in Figur 3 gezeigten zusätzlichen Druckverteilungsschicht von der Erfindung umfasst ist und eine besonders bevorzugte Ausführungsform darstellt.

Die Druckverteilungsschicht (9) kann so ausgebildet sein, dass Flüssigkeit wie Wundexsudat durch sie hindurch geleitet wird. Dazu kann die Druckverteilungsschicht geeignete Kanäle oder Öffnung enthalten. Alternativ kann sie aus einem Material bestehen, welches die Durchleitung von Wundexsudat ohne weitere Vorkehrungen zulässt.

Die zusätzliche Druckverteilungsschicht (9) kann so ausgestaltet sein, dass sie auf das Mittel zur funktionellen Verbindung des Wundraums mit einer außerhalb des Abdeckmaterials befindlichen Unterdruckquelle abgestimmt ist. Eine solche Anpassung könnte beispielsweise darin bestehen, dass die Druckverteilungsschicht(en) einen Hohlraum zur Aufnahme des Schlauchendes oder des Ports bereitstellt. Andere Anpassungen sind denkbar.

Die zusätzliche Druckverteilungsschicht kann mit dem offenzelligen Schaumstoff (c) haftend oder nicht-haftend verbunden sein.

Die Erfindung soll durch nachfolgendes Beispiel veranschaulicht werden.

### BEISPIEL

### Herstellung des offenzelligen Schaumstoffs (c)

### Teilmischung A:

60 Teile Vinylgruppen-haltiges M/D-Polysiloxan mit Viskosität von 6.000 mPas (i) 30 Teile Vinylgruppen-haltiges M/D/Q-Polysiloxan mit Viskosität von 5.500 mPas (i) 3 Teile n-Butanol (iii)
0,1 Teil Pd-Katalysator (Karstedt)

### Teilmischung B:

30 Teile Vinylgruppen-haltiges M/D-Polysiloxan mit Viskosität von 6.000 mPas (i) 10 Teile Vinylgruppen-haltiges M/D/Q-Polysiloxan mit Viskosität von 5.500 mPas (i) 30 Teile Si-H-Gruppen-haltiges M/D-Polysiloxan mit Viskosität von 4.500 mPas (iii)
Teilmischungen A und B wurden bei 22 °C in einem statischen Mischer vermischt und zur Reaktion gebracht. Nach 20 Minuten konnte der resultierende Schaumstoff entnommen und auf die gewünschte Größe geschnitten werden.

Der resultierende Schaumstoff war durch folgende Parameter gekennzeichnet:
Dichte: 0,22 g/cm3, Zugfestigkeit > 150 KPa, Bruchdehnung > 200 %, Luftdurchlässigkeit 4.000 l/(m2sec)

## Patentansprüche

1. Vorrichtung zur Unterdrucktherapie von Wunden umfassend,
(a) ein Abdeckmaterial (2) zum luftdichten Verschließen der Wunde und der Wundumgebung (1);
(b) ein Mittel (7) geeignet zur Herstellung von Unterdruck im Wundraum; und
(c) einen offenzelligen Schaumstoff (3) auf Basis eines quervernetzten Polyorganosiloxans als Wundauflage.

2. Vorrichtung zur Unterdrucktherapie von Wunden nach Anspruch 1,
wobei der Schaumstoff (c) erhältlich ist durch Umsetzung einer härtbaren Mischung umfassend die Komponenten
(i) ein Polyorganosiloxan enthaltend eine oder mehrere Gruppen mit einer C₂-C₆-Alkenylgruppe, bevorzugt enthaltend eine oder mehrere Vinyl-Gruppen,
(ii) ein Polyorganosiloxan enthaltend eine oder mehrere Si-H-Gruppen,
(iii) ein Treibmittel enthaltend eine oder mehrere OH-Gruppen, und
(iv) einen metallorganischen Katalysator.

3. Vorrichtung zur Unterdrucktherapie von Wunden nach Anspruch 2, wobei die Komponente (i) ein Polyorganosiloxan gemäß allgemeiner Formel (1) enthält, 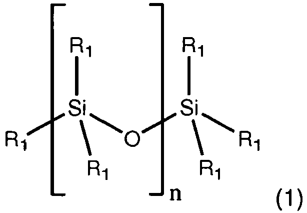 in der R¹ unabhängig C₁₋₆-Alkyl, C₁₋₆-Haloalky, Aryl oder C₂-C₆-Alkenyl ist, mit der Maßgabe dass n einen solchen Wert hat, dass die Viskosität des Polyorganosiloxans im Bereich von 500 - 250.000 mPas liegt und dass das Molekül mindestens eine C₂-C₆-Alkenylgruppe enthält.

4. Vorrichtung zur Unterdrucktherapie von Wunden nach Anspruch 2 oder 3, wobei die Komponente (ii) ein Polyorganosiloxan gemäß allgemeiner Formel (2) enthält, 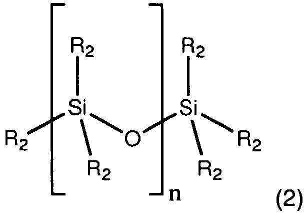 in der R² unabhängig C₁₋₆-Alkyl, C₁₋₆-Haloalky, Aryl oder Wasserstoff ist, mit der Maßgabe dass das Molekül mindestens zwei an Silicium gebundene Wasserstoffatome enthält, wobei diese an unterschiedliche Siliciumatome gebunden sind.

5. Vorrichtung zur Unterdrucktherapie von Wunden nach einem der Ansprüche 2 bis 4, wobei als Komponente (iii) ein C₁-C₆-Alkanol verwendet wird.

6. Vorrichtung zur Unterdrucktherapie von Wunden nach einem der vorangehenden Ansprüche, wobei der Schaumstoff eine Zugfestigkeit von 100 kPa bis 10 MPa, gemessen gemäß DIN 53571 und/oder eine Bruchdehnung von 100 % bis 350 %, jeweils gemessen gemäß DIN 53571, aufweist.

7. Vorrichtung zur Unterdrucktherapie von Wunden nach einem der vorangehenden Ansprüche, wobei der Schaumstoff (c) eine Luftdurchlässigkeit von 1000 bis 8000 l/(m²sec), gemessen gemäß DIN EN ISO 9237, aufweist.

8. Vorrichtung zur Unterdrucktherapie von Wunden nach einem der vorangehenden Ansprüche, wobei der Schaumstoff (c) viskoelastisches Verhalten zeigt.

9. Vorrichtung zur Unterdrucktherapie von Wunden nach einem der vorangehenden Ansprüche, wobei Schaumstoff (c) eine Rohdichte zwischen 0,12 und 0,30 kg/m³ aufweist, gemessen gemäß DIN EN ISO 845.

10. Vorrichtung zur Unterdrucktherapie von Wunden nach einem der vorangegangenen Ansprüche, wobei das Abdeckmaterial (a) eine Wasserdampfdurchlässigkeit von 100 bis 2500 g/m² x 24 h aufweist.

11. Vorrichtung zur Unterdrucktherapie von Wunden nach einem der vorangehenden Ansprüche, weiterhin umfassend mindestens eine zusätzliche Druckverteilungsschicht (9) zum Einbringen zwischen Wundauflage (c) und dem Abdeckmaterial (a).

12. Vorrichtung zur Unterdrucktherapie von Wunden nach einem der vorangehenden Ansprüche, weiterhin umfassend eine Wundkontaktschicht (10) zum Einbringen zwischen Wundoberfläche und Wundauflage (c).

13. Gebrauchsfertiges Set zur Unterdruck-Wundbehandlung umfassend,
a) Abdeckmaterial (2) zum luftdichten Verschließen der Wunde und der Wundumgebung,
b) ein Mittel, geeignet zur Herstellung von Unterdruck im Wundraum, bevorzugt ein Mittel (4-5) zur funktionellen Verbindung des Wundraums mit einer außerhalb des Abdeckmaterials (2) befindlichen Unterdruckquelle (7), so dass ein Unterdruck im Wundraum herstellbar ist und Flüssigkeiten aus dem Wundraum absaugbar sind,
c) einen offenzelligen Schaumstoff (3) auf Basis eines quervernetzten Polyorganosiloxans, wobei der Schaumstoff als Wundauflage geeignet ist und gebrauchsfertig abgepackt vorliegt.

## Claims

1. A device for negative pressure wound therapy comprising
(a) a cover material (2) for air-tight sealing the wound and the wound surroundings (1);
(b) a means (7) suitable for the generation of a negative pressure in the wound area; and
(c) an open-cell foam (3) on the basis of a cross-linked polyorganosiloxane as a wound dressing.

2. The device for negative pressure wound therapy according to claim 1, wherein the foam (c) is obtainable by the reaction of a curable mixture comprising the components
(i) a polyorganosiloxane containing one or more groups with a C₂-C₆ alkenyl group, preferably containing one or more vinyl groups,
(ii) a polyorganosiloxane containing one or more Si-H groups,
(iii) a foaming agent containing one or more OH groups, and
(iv) an organometallic catalyst.

3. The device for negative pressure wound therapy according to claim 2, wherein the component (i) contains a polyorganosiloxane in accordance with general formula (1), 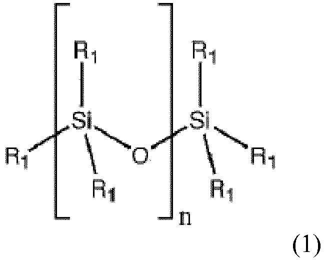 in which R¹ is independently C₁₋₆ alkyl, C₁₋₆ haloalkyl, aryl or C₂₋₆ alkenyl, provided that n has a value such that the viscosity of the polyorganosiloxane is in the range between 500 and 250.000 mPas and that the molecule contains at least one C₂₋₆ alkenyl group.

4. The device for negative pressure wound therapy according to claim 2 or 3, wherein the component (ii) contains a polyorganosiloxane in accordance with general formula (2), 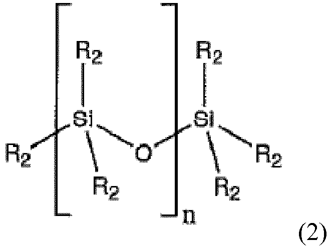 in which R² is independently C₁₋₆ alkyl, C₁₋₆ haloalkyl, aryl or hydrogen, provided that the molecule contains at least two hydrogen atoms bonded with silicon, wherein they are bonded to difference silicon atoms.

5. The device for negative pressure wound therapy according to any one of claims 2 to 4, wherein a C₁-C₆ alkanol is used as component (iii).

6. The device for negative pressure wound therapy according to any one of the preceding claims, wherein the foam has a tensile strength of 100 kPa to 10 MPa, measured in accordance with DIN 53571 and/or a ductile yield of 100% to 350%, each measured in accordance with DIN 53571.

7. The device for negative pressure wound therapy according to any one of the preceding claims, wherein the foam (c) has an air permeability of 1000 to 8000 l/(m²sec), measured in accordance with DIN EN ISO 9237.

8. The device for negative pressure wound therapy according to any one of the preceding claims, wherein the foam (c) has visco-elastic properties.

9. The device for negative pressure wound therapy according to any one the preceding claims, wherein the foam (c) has a raw density between 0.12 and 0.30 kg/m³, measured in accordance with DIN EN ISO 845.

10. The device for negative pressure wound therapy according to any one of the preceding claims, wherein the cover material (a) has a water vapor permeability of 100 to 2.500 g/m² x 24h.

11. The device for negative pressure wound therapy according to any one of the preceding claims, further comprising at least one additional pressure distribution layer (9) for insertion between the wound dressing (c) and the cover material (a).

12. The device for negative pressure wound therapy according to any one of the preceding claims, further comprising a wound contact layer (10) for insertion between the surface of the wound and the wound dressing (c).

13. A ready-to-use set for negative pressure wound therapy comprising,
a) a cover material (2) for air-tight sealing the wound and the wound surroundings,
b) a means suitable for the generation of a negative pressure in the wound area, preferably a means (4-5) for the functional connection of the wound area with a negative pressure source (7) outside of the cover material (2) in such a way that a negative pressure can be generated in the wound area and fluids can be drawn out of the wound area by suction,
c) an open-cell foam (3) on the basis of a cross linked polyorganosiloxane, wherein the foam is suitable as a wound dressing and is provided in a ready-to-use pack.

## Revendications

1. Dispositif pour la thérapie par pression négative de plaies comprenant :
(a) un matériau de recouvrement (2) pour la fermeture étanche à l'air de la plaie et des alentours de la plaie (1);
(b) un moyen (7) adapté pour la production d'une pression négative dans l'espace de la plaie; et
(c) une mousse à structure alvéolaire ouverte (3) sur la base d'un polyorganosiloxane réticulé en tant que couverture de plaie.

2. Dispositif pour la thérapie par pression négative de plaies selon la revendication 1, dans lequel la mousse (c) peut être obtenue par la mise en réaction d'un mélange durcissable comprenant les composants
(i) un polyorganosiloxane contenant un ou plusieurs groupes avec un groupe alcényle en C₂-C₆, préférablement contenant un ou plusieurs groupes vinyle,
(ii) un polyorganosiloxane contenant un ou plusieurs groupes Si-H,
(iii) un propulseur contenant un ou plusieurs groupes O-H, et
(iv) un catalyseur organométallique.

3. Dispositif pour la thérapie par pression négative de plaies selon la revendication 2, dans lequel le composant (i) contient un polyorganosiloxane selon la formule générale (1), 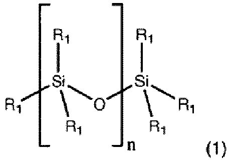 dans laquelle R¹ est indépendemment alkyle en C₁₋₆, halogéno-alkyle en C₁₋₆, aryle ou alcényle en C₂-C₆, sous la condition que n a une telle valeur, que la viscosité des polyorganosiloxanes est dans la plage de 500 - 250.000 mPas et que la molécule contient au moins un groupe alcényle en C₂-C₆.

4. Dispositif pour la thérapie par pression négative de plaies selon la revendication 2 ou 3, dans lequel le composant (ii) contient un polyorganosiloxane selon la formule générale (2), 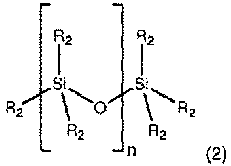 dans laquelle R² est indépendamment alkyle en C₁₋₆, halogéno-alkyle en C₁₋₆, aryle ou hydrogène, sous la condition que la molécule contient au moins deux atomes de hydrogène liés à du silicium, dans lequel ceux-ci sont liés à des atomes de silicium différents.

5. Dispositif pour la thérapie par pression négative de plaies selon une des revendications 2 à 4, dans lequel un alcanole en C₁₋₆ est utilisé en tant que composant (iii).

6. Dispositif pour la thérapie par pression négative de plaies selon une des revendications précédentes, dans lequel la mousse a une résistance à la traction de 100 kPa à 10 MPa, mesuré selon DIN 53571, et/ou un allongement à la rupture de 100 % à 350 %, mesuré respectivement selon DIN 53571.

7. Dispositif pour la thérapie par pression négative de plaies selon une des revendications précédentes, dans lequel la mousse (c) a une perméabilité à l'air de 1000 à 8000 1/(m²sec), mesuré selon DIN EN ISO 9237.

8. Dispositif pour la thérapie par pression négative de plaies selon une des revendications précédentes, dans lequel la mousse (c) montre un comportement viscoélastique.

9. Dispositif pour la thérapie par pression négative de plaies selon une des revendications précédentes, dans lequel la mousse (c) a une densité apparente entre 0,12 et 0,30 kg/m³, mesuré selon DIN EN ISO 845.

10. Dispositif pour la thérapie par pression négative de plaies selon une des revendications précédentes, dans lequel le matériau de recouvrement (a) a une perméabilité à la vapeur d'eau de 100 à 2500 g/m² x 24 h.

11. Dispositif pour la thérapie par pression négative de plaies selon une des revendications précédentes, en plus comprenant au moins une couche de dispersion de pression (9) additionnelle pour introduction entre la couverture de plaie (c) et le matériau de recouvrement (a).

12. Dispositif pour la thérapie par pression négative de plaies selon une des revendications précédentes, en plus comprenant une couche pour le contact avec une plaie (10) pour introduction entre la surface de plaie et la couverture de plaie (c).

13. Set prêt à l'emploi pour le traitement de plaies par pression négative, comprenant
(a) un matériau de recouvrement (2) pour la fermeture étanche à l'air de la plaie et des alentours de la plaie,
(b) un moyen adapté pour la production d'une pression négative dans l'espace de plaie, préférablement un moyen (4-5) pour la liaison fonctionnelle de l'espace de plaie avec une source de pression négative (7) se trouvant en dehors du matériau de recouvrement (2), de telle manière qu'une pression négative peut être produite dans l'espace de la plaie et que des liquides peuvent être aspiré à partir de l'espace de la plaie,
(c) une mousse à structure alvéolaire ouverte (3) sur la base d'un polyorganosiloxane réticulé, dans laquelle la mousse est adaptée en tant que couverture de plaie et emballée prêt à l'emploi.
